(19) Europäisches Patentamt
European Patent Office
Office européen des brevets

(11) Publication number: 0 405 104 A1

(12) **EUROPEAN PATENT APPLICATION**

(21) Application number: 90108888.0

(22) Date of filing: 11.05.90

(51) Int. Cl.5: C12P 41/00, C12P 13/00, C12P 13/04, C07C 317/00, C07D 263/14

(30) Priority: 15.05.89 US 351688

(43) Date of publication of application: 02.01.91 Bulletin 91/01

(84) Designated Contracting States: GR

(71) Applicant: SCHERING CORPORATION
2000 Galloping Hill Road
Kenilworth New Jersey 07033(US)

(72) Inventor: Clark, Jon E.
501 Grove Avenue
Highland Park, New Jersey 08904(US)
Inventor: Schumacher, Doris P.
76 Edgewood Drive
Florham Park, New Jersey 07932(US)
Inventor: Walker, Derek
38 Gloucester Road
Summit, New Jersey 07901(US)

(74) Representative: von Kreisler, Alek, Dipl.-Chem. et al
Patentanwälte Von Kreisler-Selting-Werner,
Deichmannhaus am Hauptbahnhof
D-5000 Köln 1(DE)

(54) **Process for preparing antibacterial compounds and intermediates thereto.**

(57) A process for separating enantiomer esters in a racemic mixture of the formula:

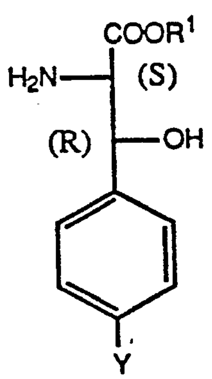

D-Threo (S,R)

and

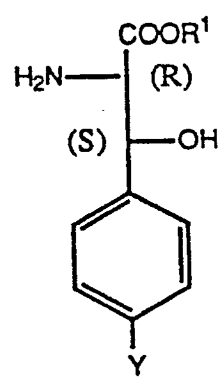

L-Threo (R,S)

(I) (II)

by hydrolyzing one of the enantiomer esters in the racemic mixture with an enzyme to its corresponding carboxylic acid.

The enzyme is a protease derived from Streptomyces griseus or Bacillus subtilies, an esterase derived from Pseudomonas cholesterol esterase or a lipase derived from the Pseudomonas genus.

The separated carboxylic acid or salt ant the substantially unhydrolyzed ester are converted to a compound of the formula:

EP 0 405 104 A1

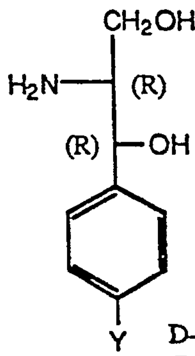

D-Threo
(R,R)

(III)

or

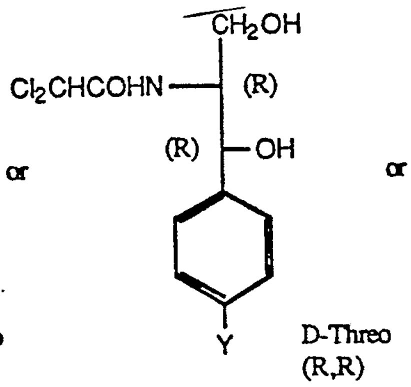

D-Threo
(R,R)

(IV)

or

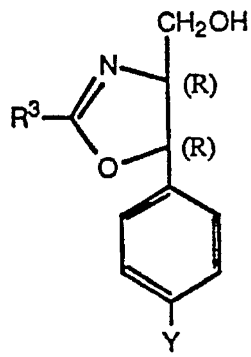

Oxazoline
(R,R)

(V)

# PROCESS FOR PREPARING ANTIBACTERIAL COMPOUNDS AND INTERMEDIATES THERETO

## Background

Thiamphenicol and florfenicol are valuable and extremely effective antibacterial compounds in world-wide use, both in the human and veterinarian fields as described in U.S. Patents 4,743,700 and 4,235,892. Thiamphenicol also serves as a starting material for preparing florfenicol. However, the relatively high cost of manufacturing thiamphenicol, and therefor florfenicol, limits their use, especially in developing nations. The high cost of manufacturing thiamphenicol is due to the present inefficient process for preparing thiamphenicol, which only partially utilizes requisite starting materials. Thiamphenicol is presently produced via the classical chemical resolution of D,L-threo 2-amino-1-(4-methylmercaptophenyl)-1,3-propanediol. The optically resolved D-threo (R,R) molecule is then simply converted to thiamphenicol by oxidation of the methylmercapto group to methylsulfonyl and by dichloroacetylation of the amine group. Unfortunately, the waste L-threo (S,S) enantiomer from the optical resolution process cannot be converted to the needed D-threo enantiomer in an economical fashion. The most recently described process for carrying out this conversion is no exception. (C. Giordano, S. Caviccholi, S. Levi and M. Villa, New Strategy for Racemisation of 2-Amino-1,3-propanediols, Key intermediates for the synthesis of Antibiotic Drugs, Tetrahedron Letters, Vol. 29, No. 43, pp. 5561-5564, (1988).) The Giordano et al. process requires many steps, affords low yields and is too expensive to adopt on a commercial scale. Thus, at least half of the raw materials and labor needed to produce thiamphenicol are discarded as waste, adding environmental concerns to the cost problem.

One solution to the problem would be to resolve the precursor to D,L-threo-2-amino-1-(4-methylmercaptophenyl)-1,3-propanediol, namely the methyl or ethyl esters of D, L-threo-4-methylmercaptophenylserine. Unfortunately to date, no effective method has been found for carrying out this resolution. If this separation could be achieved there is good literature indication (Japan Kokai 75,148,326 27 November 1975, Appl. 74 49 370, 01 May 1974) that L-threo esters of 4-methylsulfonylphenylserinate, which should be obtainable from L-threo esters of 4-methylmercaptophenylserine, could be converted to the desired D-threo compound. In short, the missing component needed to create an economical manufacturing process for thiamphenicol, and thence florfenicol, is the efficient separation of esters of the D and L threo isomers of 4-methylmercaptophenylserine.

There is therefore a need to find a process which reduces the cost of manufacture of antibacterial compounds such as thiamphenicol, which almost entirely eliminates the waste presently associated with their manufacture. Specifically, it would be desirable to provide a process which can easily and economically separate (S,R) and (R,S) i.e. D-and L-threo enantiomers from a racemic mixture, such as D,L-Threo-4-methylmercaptophenylserine, in such a way as to provide each enantiomer in a highly optically pure form.

## SUMMARY OF THE INVENTION

We have now discovered that certain enzymes can selectively hydrolyze one enantiomer ester in a racemic mixture, and thus enable the desired separation of D,L-threo (S,R and R,S) esters.

In its first embodiment, the present invention is directed toward a process for separating enantiomer esters in a racemic mixture of the formula:

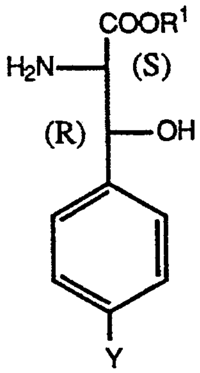

D-Threo
(S,R)

and

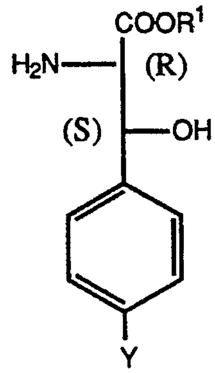

L-Threo
(R,S)

(I)

(II)

wherein $R^1$ is alkyl, substituted alkyl, alkenyl, substituted alkenyl, aryl, substituted aryl, aralkyl, substituted aralkyl, aromatic heterocyclic, substituted aromatic heterocyclic, heterocyclic alkyl or substituted heterocylic alkyl; and

Y is -H, $-NO_2$, $-S-R^2$ wherein $R^2$ is alkyl, $-SO-R^2$ or $-SO_2R^2$

comprising:

(a) selectively hydrolyzing one of the enantiomer esters in the racemic mixture with an enzyme to its corresponding carboxylic acid or salt having an enantiomeric purity of about 80 percent or greater, while leaving the other enantiomer ester substantially unhydrolyzed; and

(b) separating the carboxylic acid or salt compound from the unhydrolyzed enantiomer ester.

In a second embodiment, the present invention further comprises:

c) converting both the separated carboxylic acid or salt and the substantially unhydrolyzed ester into a compound of the formula:

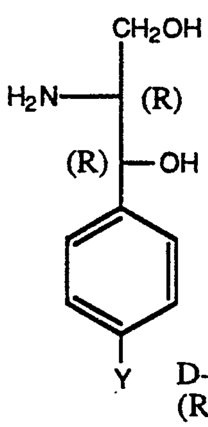

D-Threo
(R,R)

(III)

or

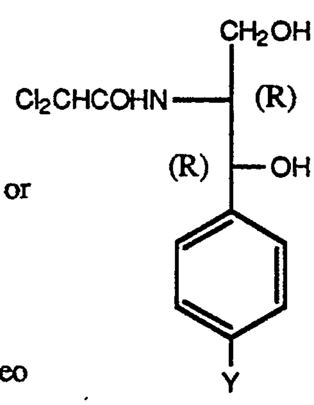

D-Threo
(R,R)

(IV)

or

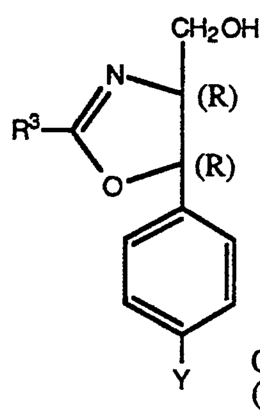

Oxazoline
(R,R)

(V)

wherein Y is as defined hereinbefore and $R^3$ is H, alkyl, substituted alkyl, alkenyl, substituted alkenyl, aryl, substituted aryl, aralkyl, substituted aralkyl, aromatic heterocyclic, substituted aromatic heterocyclic, heterocyclic alkyl or substituted heterocylic alkyl.

## EMBODIMENTS OF THE INVENTION

The term "alkyl" refers to a straight chain saturated hydrocarbon moiety containing from 1 to 6 carbon atoms, or a branched saturated hydrocarbon moiety of 3 to 6 carbon atoms, such as for example, methyl (ie. $-CH_3$), ethyl, propyl, isopropyl, butyl, isobutyl, tert-butyl, pentyl, hexyl and the like.

The term "alkenyl" refers to a straight hydrocarbon moiety of two to six carbon atoms or a branched

hydrocarbon moiety of two to six carbon atoms having at least one carbon to carbon double bond such as allyl, ethenyl, propenyl, 1-butenyl, 2-butenyl, isobutenyl, 1-pentenyl, 2-methyl-1-butenyl, 1-hexenyl and the like.

The term "aryl" refers to a carbocyclic moiety containing at least one benzenoid-type ring, with the aryl groups preferably containing from 6 to 15 carbon atoms, for example, phenyl, naphthyl, indenyl, indanyl and the like.

The term "aralkyl" refers to an aryl moiety of six to 15 carbon atoms covalently bonded to an alkyl moiety of one to six carbon atoms such as, for example, benzyl, phenyethyl, and the like.

The term "aromatic heterocyclic" refers to a cyclic moiety having at least one oxygen (O), sulfur (S) and/or nitrogen (N) heteroatom interrupting the ring structure and having a sufficient number of unsaturated carbon to carbon bonds, nitrogen to carbon bonds, and the like, to provide aromatic character, with the aromatic heterocyclic groups preferably containing from 2 to 14 carbon atoms, for example, 2-, 3- or 4-pyridyl, 2- or 3-furyl, 2- or 3-thienyl, 2-, 4- or 5-thiazolyl, 2-, 4- or 5-imidazolyl, 2-, 4- or 5-pyrimidinyl, 2-pyrazinyl, 3- or 4-pyridazinyl, 3-,5-or 6[1,2,4-triazinyl], 3- or 5-[1,2,4-thiadiazolyl], 2-,3-, 4-, 5-, 6- or 7-benzofuranyl, 2-, 3-, 4-, 5-, 6- or 7- indolyl, 3-, 4- or 5-pyrazolyl, 2-, 4- or 5-oxazolyl, and the like.

The term "heterocyclic alkyl" refers to an aromatic heterocyclic moiety of 2 to 14 carbon atoms as defined hereinbefore, covalently bonded to an alkyl moiety of one to six carbon atoms.

The terms "halogen" and "halo" refer to fluoride, chloride, bromide or iodide.

The terms "substituted alkyl", "substituted alkenyl", "substituted aryl", "substituted aralkyl", "substituted aromatic heterocyclic" and "substituted heterocyclic alkyl" refer to the above corresponding species which are further substituted at the carbon or heteroatom by one or more of the following groups: halo, alkyl of one to six carbon atoms, aryl of six to fifteen carbon atoms, cyano (ie. -CN), carboxyl (ie. -COOH) or salts thereof, nitro (ie. $-NO_2$) and hydroxyl.

The term "enantiomeric purity" refers to the amount of one enantiomer in the hydrolyzed carboxylic acid. As described in The Vocabulary of Organic Chemistry, Milton Orchin, Fred Kaplan, Roger S. Macomber, R. Marshall Wilson and Hans Zimmer, Wiley-Interscience John Wiley and Sons, New York, 1980, pp 130 an enantiomeric purity of 100 percent indicates that the hydrolyzed species from a racemic mixture is 100 percent one enantiomer. An enantiomeric purity of 80 percent indicates that the hydrolyzed species from a racemic mixture is 80 percent one enantiomer and 20 percent the other enantiomer. Likewise, an enantiomeric purity of 50 percent indicates that both enantiomers in a racemic mixture are equally hydrolyzed ie. there is no selectivity.

The term "enzyme" is intended to mean in its broadest sense, an organic catalyst produced by living cells but capable upon separation from the cell or upon chemical modification, of acting independently.

The term "protease" indicates protein-splitting enzyme.

The term "lipase" indicates lipid-splitting enzyme.

The term "esterase" indicates ester-splitting enzyme.

As to enantiomer esters I or II, preferably $R^1$ is methyl, ethyl or isopropyl, more preferably $R^1$ is ethyl or isopropyl. Also preferred is that Y is $-SR^2$, more preferably wherein $R^2$ is methyl.

The enantiomer ester selectively hydrolyzed to the carboxylic acid or salt may be either the D-Threo (S,R) or L-Threo (R,S) ester of formula I or II, more preferably the D-Threo ester. Enzymes for selectively hydrolyzing compounds of formulas I or II are available from commercial suppliers such as the Sigma Chemical Co., St. Louis, Missouri. Such enzymes include but are not limited to:

| Enzyme | Source |
|---|---|
| Protease | Streptomyces griseus |
| Protease | Bacillus subtilis |
| | Aspergillus oryzae |
| | Streptomyces caespitosus |
| Lipase | Pseudomonas |
| Esterase | Pseudomonas cholesterol esterase |

In step (a), enantiomer esters I and II in the racemic mixture are distinguished by selectively hydrolyzing one of the enantiomer esters, i.e. I or II to its corresponding carboxylic acid or salt with one of the above enzymes, and leaving the other enantiomer substantially unhydrolyzed. The carboxylic acid or salt is then separated by base extraction or other means for separation such as chromatography. The particular ester which is selectively hydrolyzed should be hydrolyzed to an enantiomeric purity of about 80

percent (%) or greater. More preferably, the enzyme should be able to selectively hydrolyze one of the enantiomer esters to its corresponding carboxylic acid or salt in an enantiomeric purity of about 90 percent or greater, even more preferably about 94 percent or greater, most preferably about 97 percent or greater. The enzyme can be in free or immobilized form, preferably immobilized. Methods for immobilizing such enzymes can be found in Methods of Enzymology, Volume XLIV, Ed. by Klaus Mosbach, Academic Press, New York, 1976.

The racemic mixture is contacted with a suitable enzyme in amounts and under conditions effective to selectively hydrolyze one of the enantiomer esters. In a batch procedure the amount of enzyme used in the present method can range from about 1 g to about 1 µg of enzyme per one gram of enantiomer esters I and II, preferably from about 0.01 g to about 0.5 g of enzyme per gram of enantiomer ester. However, where an immobilized enzyme is employed, the amount of enzyme can be less than the amounts described above. The contacting can be carried out in an aqueous medium, preferably distilled or deionized water, or water mixed with a suitable miscible or immiscible organic solvent. Suitable organic solvents include but are not limited to $C_1$ to $C_6$ alcohols, such as methanol and ethanol, dihydric or trihydric alcohols such as glycol or glycerol, or other solvents such as dimethylsulfoxide, acetonitrile, dimethylformamide, dimethylacetamide, acetone, methyl ethyl ketone and tetrahydrofuran, toluene, hexane, tert-butyl methyl ether, methylethyl ketone; methyl isobutyl ketone, methylene chloride, or mixtures of any of the above. The organic solvents can be mixed with water in concentrations up to 50% by volume or more but preferably up to about 10% by volume.

Optionally, the aqueous medium can contain a suitable buffer, such as those described in Gordon and Ford (Ed.) The Chemist's Companion: A Handbook of Practical Data, Techniques and References, pp. 71 (1972), whose preparative teachings are incorporated herein by reference. The aqueous medium is maintained between a pH of about 4 to 9, preferably a pH of about 5.5 to 8, more preferably a pH of about 5.5 to 7. Any buffering agent used can be employed in amounts ranging from about zero to about one mole/liter (M), preferably about 0.05M. Alternatively, a suitable base can be added to neutralize the acid produced during hydrolysis. Suitable bases include sodium and potassium hydroxides, carbonates, bicarbonates and mixtures thereof.

The selective hydrolysis of one of the enantiomer esters in step (a) can be carried out at temperatures ranging from about -5 degrees Centigrade (°C) to about 80°C, preferably from about 10 to about 45°C, more preferably from about 30 to about 35°C. The reactants are preferably stirred for a time effective to selectively hydrolyze one of the enantiomer esters to its corresponding carboxylic acid or salt, ranging from about 30 minutes to about 48 hours or more until the reaction is completed to the extent desired.

In step (b) the substantially unhydrolyzed enantiomer ester can be recovered from the reaction mixture by extracting the aqueous layer with a suitable water-immiscible solvent at a pH of about 8 or greater. Such water-immiscible solvents include but are not limited to the chlorinated hydrocarbons such as chloroform, carbon tetrachloride, and methylene chloride, the alkyl esters such as ethyl acetate, isopropyl acetate and ethyl benzoate, the aromatic hydrocarbons such as toluene and xylenes, and ethers such as tert-butylmethyl ether (tert-BuOMe). Following extraction, the water-immiscible solvent can be removed by conventional procedures such as distillation, and the like to give the unhydrolyzed enantiomer ester.

The remaining alkaline aqueous layer can be acidified, filtered and concentrated by conventional procedures such as lyophilization, distillation, chromatography and the like to give the desired carboxylic acid or salt.

Alternatively, the carboxylic acid or salt may be separated from the unhydrolyzed ester employing chromatographic procedures such as described in Snyder and Kirkland, Introduction to Modern Liquid Chromatography, 2nd ed., Wiley-Interscience, New York, 1979. For example, after selective hydrolysis of the enantiomer ester, the reaction mixture can be chromatographed over gravity based or normal phase silica gel columns using suitable eluting solvents.

The separated carboxylic acid or salt and the substantially unhydrolyzed enantiomer ester can be converted to desired compounds III, V and IV according to the following scheme:

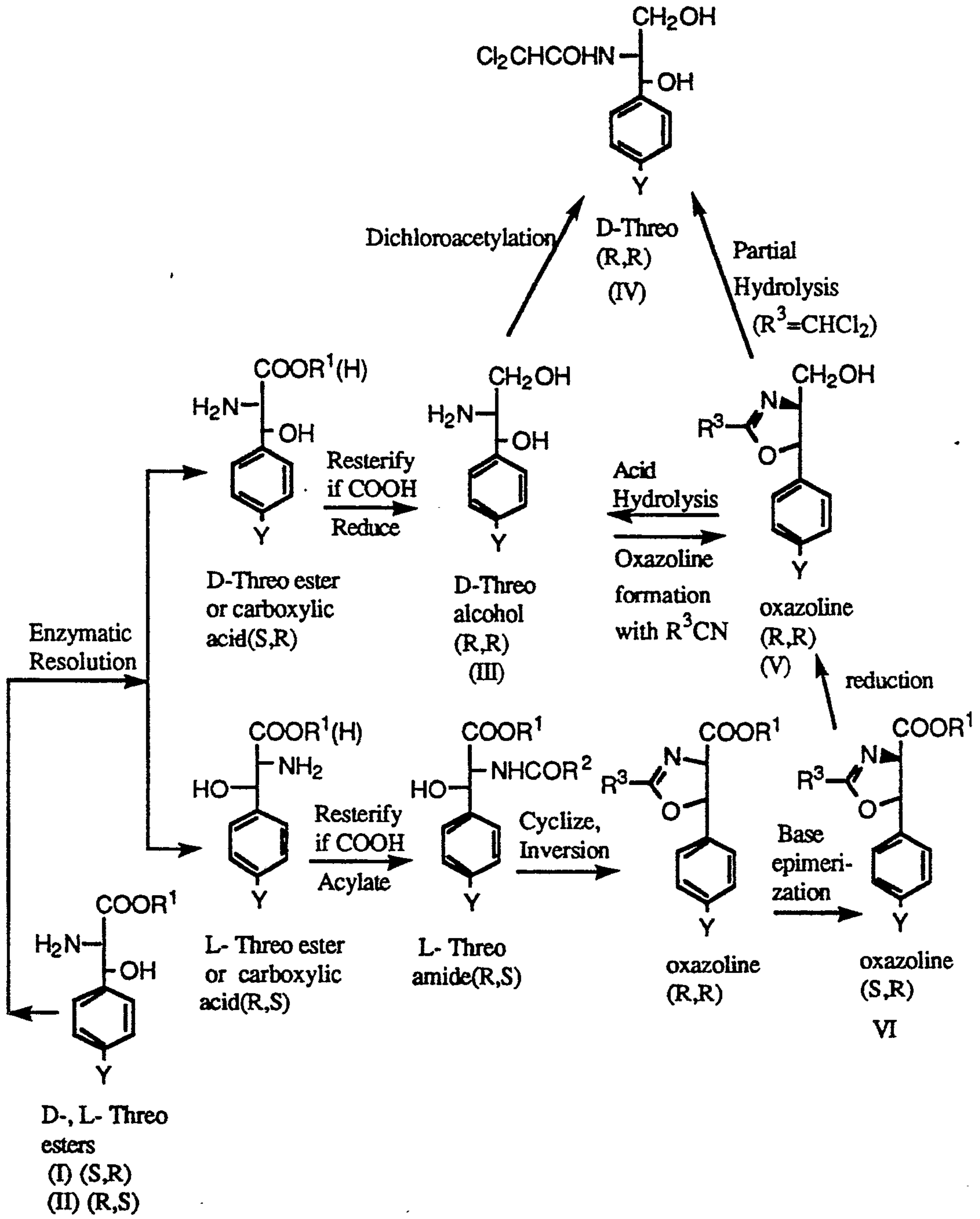


After the carboxylic acid or salt is separated from the unhydrolyzed enantiomer ester, the carboxylic acid can be reesterified to any ester desired using conventional esterification procedures, such as described in Jerry March, Advanced Organic Chemistry, Reactions, Mechanisms and Structure, John Wiley and Sons, New York, New York, 1985, 1346 pp., whose preparative teachings are incorporated herein by reference.

The D-threo ester(S,R) or reesterified D-threo carboxylic acid can be converted to the D-Threo alcohol (R,R) of formula III by reducing the ester moiety of the D-Threo ester using known reducing agents and under conditions such as described in March, supra. Suitable reducing agents include $LiAlH_4$, $LiBH_4$, $Ca(BH_4)_2$ or $Ca(BH_4)_2$ prepared from $CaCl_2$ and $NaBH_4$ Alternatively, the ester can be reduced with a hydrogenating agent, including the requisite hydrogenating catalyst(s) and hydrogen. Various selected catalysts and conditions are described in "Catalytic Hydrogenation in Organic Synthesis", (1978) Morris Freifelder, Chapter 4, Olefins, pages 15-25, John Wiley and Sons and in March, supra. The hydrogenating catalyst can be nickel, palladium, platinum, platinum oxide, platinum on carbon, and mixtures thereof. The source of hydrogen can be hydrogen gas or isotopic forms thereof, such as deuterium or tritium.

The dichloro D-threo (R,R) of formula IV can be prepared by dichloroacetylation with a $C_1$ to $C_6$ ester of dichloroacetic acid, dichloroacetyl chloride or dichloroacetic anhydride. Preferably the $C_1$ to $C_6$ ester of dichloroacetic acid is the methyl or ethyl ester. The D-threo (R,R) of Formula IV wherein Y is $-SO_2CH_3$ and the amine has been acylated to the dichloroacetamide (i.e. $-NHCOCHCl_2$) is known as thiamphenicol.

The L-threo ester (R,S) or re-esterfied carboxylic acid (R,S) can be converted to the D-threo of formulas III, V or IV by procedures similar to those described in Japan Kokai 75,148,326 27 November 1975, Appl 74 49 370, 01 May 1974. Essentially, the L-threo ester (R,S) is acylated with conventional acylating agents to give the L-threoamide (R,S). The L-threoamide (R,S) is treated with a Lewis acid to form a leaving group with the benzylic carbinol and to induce cyclisation with inversion of the stereoisomeric center, forming the oxazoline (R,R). Suitable Lewis acids for the melted cyclization with inversion of L-threo (R,S) include but are not limited to thionyl chloride, phosphorus trichloride, phosphorus oxychloride, boron trichloride and phosphorus pentachloride, preferably thionyl chloride.

The oxazoline (R,R) thus prepared can be treated with a base effective to cause base epimerization of the oxazoline (R,R) ester to the oxazoline (S,R) of formula VI. The base should be strong enough to abstract the proton from the carbon atom connected to the nitrogen atom. Suitable bases include sodium methylate, sodium ethylate, Hunig's base, tetramethylguanidine or triethylenediamine, commercially available as DABCO®, trademark of Air Products and Chemicals, Inc. Allentown, Pennsylvania. Preferably, the base is sodium ethylate.

The oxazoline ester VI can be converted to the corresponding oxazoline carbinol of formula V according to the same reduction procedures as described above for converting the D-Threo (S,R) ester to the (R,R) D-Threo alcohol.

The oxazoline (R,R) of formula V can also be prepared by contacting the D-Threo alcohol (R,R) of formula III with a cyano compound of the formula $R^3CN$ wherein $R^3$ is as defined herein, in the presence of a base and an alcoholic solvent under conditions effective to yield the oxazoline (R,R) of formula V, such as described in U.S. Patent Application Serial No. 244,126 filed September 14, 1988 whose preparative teaching are incorporated herein by reference.

The oxazoline (R,R) of Formula V (wherein $R^3$ is dichloromethyl) prepared by either of the above two methods can be partially hydrolyzed with acid to yield the dichloro D-threo (R,R) of Formula IV. The hydrolysis can be performed at pH less than 5, preferably at pH less than 1 at temperatures ranging from about ambient to about 95°C. Suitable acids for hydrolysis include hydrochloric and sulfuric acids.

Alternatively, the oxazoline (R,R) of Formula V can be fully hydrolyzed with the above acids by use of higher temperatures or longer times than those used for partial hydrolysis to give the D-threo aminodiol (R,R) of Formula III. This compound can then be converted to the compound of formula IV by dichloroacetylation of the amine.

It can thus be appreciated that compounds III, IV or V prepared from both the separated D-threo (S,R) and L-threo (R,S) compounds can be combined to efficiently utilize each enantiomer starting material in the racemic mixture.

## Preparation of starting materials

The D-Threo(S,R) and L-Threo(R,S) compound of formulas (I) and (II) are known to those skilled in the art, as taught in Giordano et al. and Japan Kokai 75,148,326 supra. The esters of such compounds can be prepared by known esterification procedures, such as those described in March, supra.

The following examples illustrate the present invention in a manner by which it can be practiced, but as such, should not be construed as limitations upon the overall scope of the same.

## EXAMPLE 1

### Enzymatic Resolution of methyl D,L-threo-4-methylmercaptophenylserinate

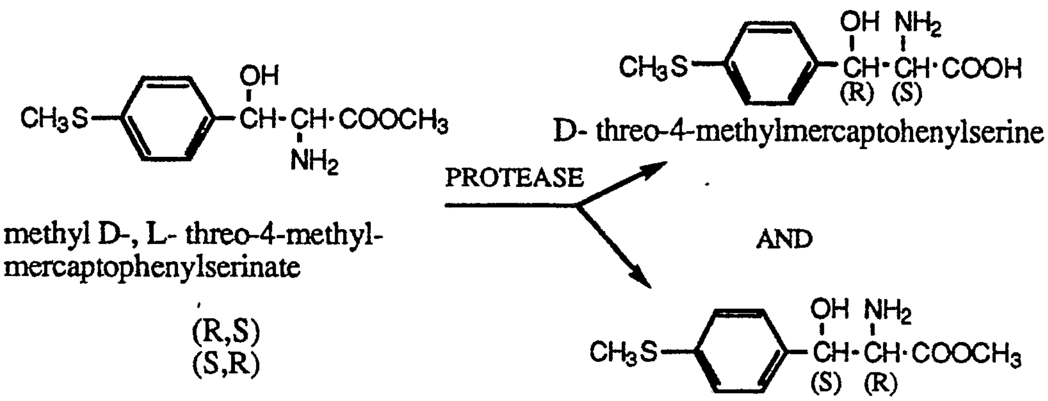


D- threo-4-methylmercaptohenylserine

methyl D-, L- threo-4-methyl-mercaptophenylserinate

(R,S)
(S,R)

AND

methyl L- threo-4-methylmercaptophenyl-serinate

A solution of one percent (volume basis) DMSO in water at 45°C is passed through a 15 milliliters (ml) packed bed of immobilized protease derived from Streptomyces griseus on sepharose, (Sigma catalog number P4531) at a flow rate of 2.2 milliliters per minute (ml/min). A solution of 3.0 grams (g), of methyl D, L-threo-4-methylmercaptophenylserinate, in 15 ml of DMSO is pumped into the aqueous flow stream, before the column, at a flow rate of 0.01 ml/min. The column effluent is dripped into stirred ethyl acetate (EtOAC) maintained at a temperature of -40°C to give a slurry of ice. Once the desired hydrolysis is completed the ice is melted and adjusted to pH 8 using 50 percent NaOH. The organic layer is separated and the aqueous is extracted six times using methylene chloride ($CH_2Cl_2$). The organic extracts are combined, dried over sodium sulfate and adjusted to pH 1.0 using methanol saturated with hydrogen chloride, concentrated to a residue and maintained under 10 millimeters mercury pressure for 24 hours. The residue is dissolved in water and washed three times with $CH_2Cl_2$. The aqueous layer is then adjusted to pH 8 and extracted three times with $CH_2Cl_2$. The $CH_2Cl_2$ extracts are combined and dried over $Na_2SO_4$ and adjusted to pH 1.0 using methanol saturated with hydrogen chloride and concentrated to a dry residue to give 1.3 g of methyl L-threo-4-methylmercaptophenyl serinate hydrochloride having an enantiomeric purity of 99 percent. The aqueous layer is lyophilized (i.e. freeze dried) to give 1.2 g of D-threo-4-methylmercaptophenyl serine having an enantiomeric purity of 97.5 percent (88 percent yield).

Following essentially the same procedure as described in Example 1, the following results as described in Table 1 are obtained.

## Table 1

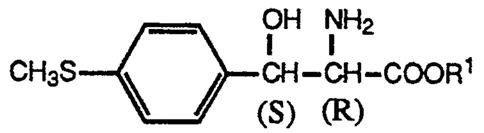

(R,S)

| Enzyme | Source | $R^1$ | Enantiomeric Purity (%) |
|---|---|---|---|
| Protease | Streptomyces griseus | methyl | 99.0 |
| Protease | Streptomyces griseus | isopropyl | 97.5 |
| Protease | Bacillus subtilis VIII | isopropyl | 91.5 |
| Protease | Bacillus subtilis XVI | isopropyl | 91.0 |
| Protease | Bacillus subtilis XXIV | isopropyl | 92.0 |
| Protease | Bacillus subtilis XXVII | isopropyl | 90.6 |
| Protease | Aspergillus oryzae XXIII | isopropyl | 87.0 |
| Protease | Streptomyces caespitosus | isopropyl | 81.0 |

The following preparative examples are intended to represent various methods by which starting materials employed in the present process can be prepared, but should not be construed as limitations upon the overall scope of the same.

### PREPARATIVE EXAMPLE 1

D,L-Threo-4-Methylmercaptophenylserine, a racemic mixture of (R,S) and (S,R) enantiomers

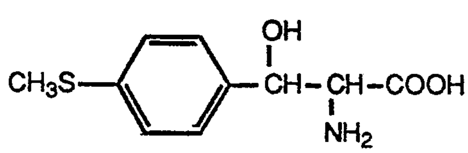

(R,S)
(S,R)

To a solution of 101 g of glycine in 570 mL of water, add 94 g of sodium hydroxide followed by 570 g of 4-methylmercaptobenzaldehyde. Stir the mixture at 75° C for 1.5 hours. Add 115 mL of ethanol and stir at room temperature overnight. Cool the mixture to 5° C and add 570 mL of hydrochloric acid. Extract the

solution twice with tert-butylmethyl ether to remove excess aldehyde. Concentrate the water layer, adjust to pH 3 with ammonium hydroxide and cool to 5 ° C. Collect the precipitate by filtration, wash with water and dry under vacuum. Triturate the solids with methanol, filter and dry under vacuum to give 178 g of title compound, a solid (Yield 58%).

## PREPARATIVE EXAMPLE 2

Methyl D, L-threo-4-Methylmercaptophenylserinate, a racemic mixture of (R,S) and (S,R) enantiomers

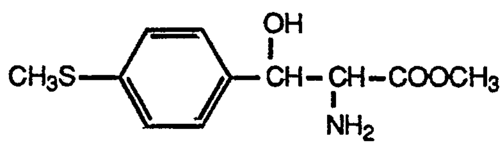

(R,S)
(S,R)

A solution is prepared by adding 250 mL of thionyl chloride ($SOCl_2$) to one liter (L) of methanol ($CH_3OH$) at -25 ° C. To this solution is added 220 g D,L-threo-4-methylmercaptophenylserine. The reaction is allowed to warm to room temperature with stirring. The mixture is stirred for three days and then concentrated to a residual oil. The oil is dissolved in 2 liters (L) toluene and 200 mL triethylamine is added. The solids are removed by filtration and the mixture is concentrated to a slurry. The crystals are collected by filtration and washed with toluene and dried in vacuum to give 174 g of the title compound, a solid (66 % yield).

## PREPARATIVE EXAMPLE 3

Ethyl D,L-Threo-4-Methylmercaptophenylserinate

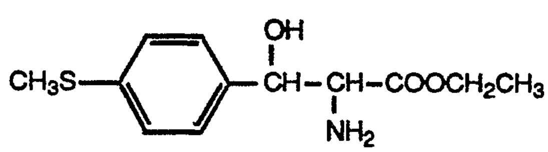

(R,S)
(S,R)

A slurry of 50 g of D, L-threo-4-methylmercaptophenylserine in 500 mL of ethanol is saturated with hydrogen chloride. This mixture is stirred at room temperature for three days. The solids are removed by filtration through Celite® diatomaceous earth, trademark of Johns-Manville Products Corporation, Manville, New Jersey. The filtrate is concentrated to 200 mL and filtered again. This solution is concentrated to 75 mL and cooled to 0 ° C. The solids are collected by filtration and washed with ice cold ethanol. The solids are redissolved in clean ethanol and the pH is adjusted to pH 1.0 using ethanol saturated with hydrogen chloride. This is concentrated to a slurry, cooled to 0 ° C and the solids collected by filtration and washed

with diethylether, then dried in vacuum. The solids are dissolved in 200 mL water and washed with 200 mL $CH_2Cl_2$. The pH of the aqueous is adjusted to pH 9 using triethylamine and extracted six times using 200 mL $CH_2Cl_2$. The combined $CH_2Cl_2$ extracts are dried using $Na_2SO_4$, filtered and concentrated to a residue. The solids are recrystallized by concentrating a solution in ethanol to a slurry, maintaining the temperature below 40° C. The solids are collected at 0° C and washed with cold ethanol and dried in vacuum to give 30g of title compound, a solid (53% yield).

PREPARATIVE EXAMPLE 4

Isopropyl D,L-threo-4-Methylmercaptophenylserinate-HCl, a racemic mixture of (R,S) and (S,R) enantiomers

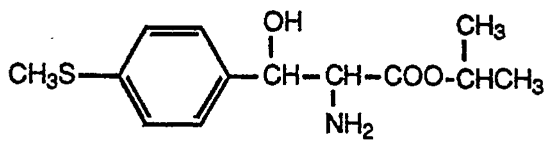

(R,S)
(S,R)

A slurry of 16 g of D,L-threo-4-methylmercaptophenylserine in 1.5L isopropanol is heated to reflux. A stream of hydrogen chloride gas is bubbled through the refluxing slurry until a clear yellow solution is formed. The reflux and the gas stream are continued for 4 hours. While the gas stream is continued, the isopropanol is removed by distillation to a volume of 100 mL. This is then cooled to 50° C and 1.5 L of isopropyl acetate is added. This mixture is stirred at 0° C for 2 hours after which the crystals are collected by filtration and washed with cold isopropyl acetate and then with cold diethylether. The crystals are dried in vacuum overnight to give 11.4 g of title compound, a solid (53% yield).

While the present invention has been described in conjunction with the specific embodiments set forth above, many alternatives, modifications, and variations thereof will be apparent to those of ordinary skill in the art. All such alternatives, modifications and variations are intended to fall within the spirit and scope of the present invention.

## Claims

1. A process for separating enantiomer esters in a racemic mixture of the formula:

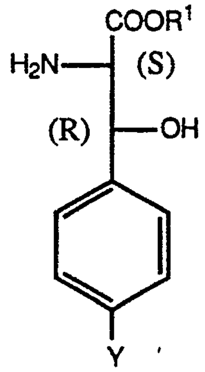

D-Threo
(S,R)

(I)

and

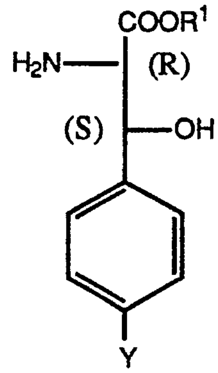

L-Threo
(R,S)

(II)

wherein $R^1$ is alkyl, substituted alkyl, alkenyl, substituted alkenyl, aryl, substituted aryl, aralkyl, substituted aralkyl, aromatic heterocyclic, substituted aromatic heterocyclic, heterocyclic alkyl or substituted heterocylic alkyl; and

Y is -H, $-NO_2$, $-S-R^2$ wherein $R^2$ is alkyl, $-SO-R^2$ or $-SO_2R^2$

comprising:

(a) selectively hydrolyzing one of the enantiomer esters in the racemic mixture with an enzyme to its corresponding carboxylic acid or salt having an enantiomeric purity of about 80 percent or greater, while leaving the other enantiomer ester substantially unhydrolyzed; and

(b) separating the carboxylic acid or salt from the substantially unhydrolyzed enantiomer ester.

2. The process of claim 1 wherein one of the enantiomer esters is hydrolyzed to an enantiomer purity of about 90 percent or greater.

3. The process of claims 1-2 wherein one of the enantiomer esters is hydrolyzed to an enantiomer purity of about 97 percent or greater.

4. The process of claims 1-3 wherein the enzyme is a protease, a lipase or an esterase.

5. The process of claims 1-4 wherein the enantiomer ester selectively hydrolyzed is the (S,R) enantiomer ester of formula I.

6. The process of claims 1-5 wherein the enzyme is derived from one of the following genus: Streptomyces, Bacillus or Pseudomonas.

7. A process of claims 1-6 wherein the enzyme is a protease derived from Streptomyces griseus or Bacillus subtilies, an esterase derived from Pseudomonas cholesterol esterase or a lipase derived from the Pseudomonas genus.

8. The process of claims 1-7 wherein the enzymatic hydrolysis is carried out in a pH range of about 5.5 to 7.

9. The process of claims 1-8 wherein in step (b) the carboxylic acid or salt is separated from the substantially unhydrolyzed ester by extracting the water with an immiscible organic solvent, and separating the water layer containing the carboxylic acid or salt from the immiscible organic solvent containing the substantially unhydrolyzed ester.

10. The process of claims 1-9 wherein $R^1$ is methyl and Y is $-SCH_3$.

11. The process of claims 1-10 further comprising step (c), converting both the separated carboxylic acid or salt and the substantially unhydrolyzed ester to a compound of the formula:

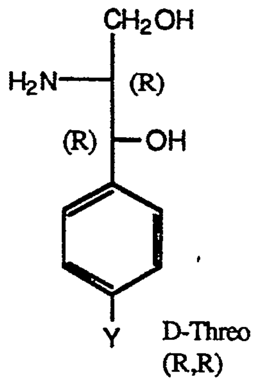

D-Threo
(R,R)
(III)

or

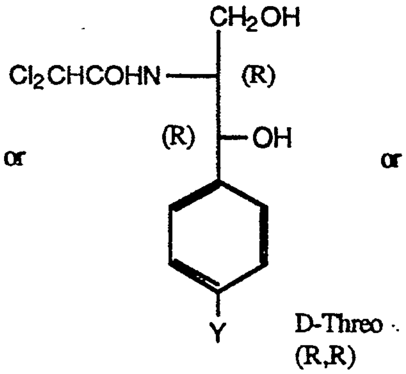

D-Threo
(R,R)
(IV)

or

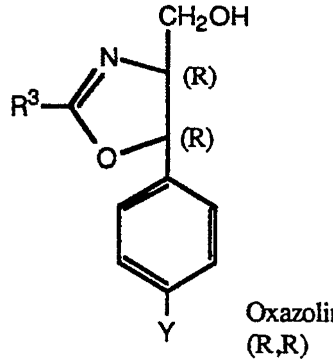

Oxazoline
(R,R)
(V)

wherein Y is as defined hereinbefore; and $R^3$ is H, alkyl, substituted alkyl, alkenyl, substituted alkenyl, aryl, substituted aryl, aralkyl, substituted aralkyl, aromatic heterocyclic, substituted aromatic heterocyclic, heterocyclic alkyl or substituted heterocylic alkyl.

European Patent Office

# EUROPEAN SEARCH REPORT

Application Number

EP 90 10 8888

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 141 613 (ZAIDAN)<br>* Claims * | 1 | C 12 P 41/00<br>C 12 P 13/00<br>C 12 P 13/04<br>C 07 C 317/00<br>C 07 D 263/14 |
| Y | US-A-3 347 752 (E. RAUENBUSCH)<br>* Claims * | 1 | |
| Y | CHEMICAL ABSTRACTS, vol. 105, no. 3, 21st July 1986, page 537, abstract no. 23102k, Columbus, Ohio, US; & JP-A-60 248 192 (SUMITOMO CHEMICAL CO., LTD) 07-12-1985 | 1 | |
| Y | FR-A-2 054 852 (SUMITOMO)<br>* Claims * | 1,11 | |
| Y | CHEMICAL ABSTRACTS, vol. 85, no. 3, 19th July 1976, page 736, abstract no. 21829x, Columbus, Ohio, US; & JP-A-75 148 326 (SHIZUOKA CAFFEINE INDUSTRY CO., LTD) 27-11-1975 | 1 | TECHNICAL FIELDS SEARCHED (Int. Cl.5) |
| Y | EP-A-0 130 633 (ZAMBON)<br>* Claims * & US-A-4 743 700 (Cat. D) | 1,11 | C 12 P<br>C 07 C<br>C 07 D |

The present search report has been drawn up for all claims

| Place of search | Date of completion of the search | Examiner |
|---|---|---|
| THE HAGUE | 19-09-1990 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document